# EUROPÄISCHE PATENTSCHRIFT

(11) **EP 1 811 300 B1**
(45) Veröffentlichungstag und Bekanntmachung des Hinweises auf die Patenterteilung: **12.11.2008**
(21) Anmeldenummer: 05028400.9
(22) Anmeldetag: 23.12.2005
(51) Int. Cl.: G01N 33/543, G01N 33/553

(54) **Sensorchip mit in einem Polymer-Netzwerk eingelagerten Rezeptoren**
Sensor chip with receptors included in a polymeric network
Puce de capteur avec des récepteurs inclus dans un réseau polymérique

(43) Veröffentlichungstag der Anmeldung: 25.07.2007
(73) Patentinhaber: Micronas GmbH, 79108 Freiburg i. Br. (DE); Micronas Holding GmbH, 79108 Freiburg i.Br. (DE)
(72) Erfinder: Freund, Ingo, Dipl.-Ing. (FH), 79117 Freiburg (DE); Lehmann, Mirko, Dr. rer. nat, 79117 Freiburg (DE); Mohry, Sonja, 79108 Freiburg (DE); Baader, Johannes, 79102 Freiburg (DE); Klapproth, Holger, Dr., 79108 Freiburg (DE)
(74) Vertreter: Huwer, Andreas

(56) Entgegenhaltungen:
- EP-A- 1 202 062
- EP-A- 1 496 363
- WO-A-02/066162
- DE-A1- 10 201 653
- US-A1- 2003 099 949
- "WIKIPEDIA - die freie Enzyklopädie" [Online] Gefunden im Internet: URL:http://de.wikipedia.org/wiki/Polyethyl englykol>

## Beschreibung

Die Erfindung betrifft einen Sensor-Chip, mit einem Träger, an dessen Oberfläche Teststellen vorgesehen sind, an denen Rezeptoren immobilisiert sind, die für einen zu detektierenden Liganden bindungsspezifisch sind, wobei die Rezeptoren in einer Monolage und/oder in mehreren übereinander geschichteten Moleküllagen an die Oberfläche gebunden sind. Femer betrifft die Erfindung einen Sensor-Chip, mit einem Träger, an dessen Oberfläche mindestens eine Teststelle vorgesehen ist, an der ein Polymer-Netzwerk fixiert ist, auf und/oder In dem Rezeptoren immobilisiert sind, die für einen zu detektierenden Liganden blndungsspeziflsch sind. Außerdem betrifft die Erfindung ein Verfahren zur Messung einer ersten Konzentration eines in einem zu untersuchenden Analyten enthaltenen ersten Liganden und einer größeren zweiten Konzentration eines in dem Analyten enthaltenen zweiten Liganden.

Ein derartiger Sensor-Chip mit einem Träger, der an seiner Oberfläche ein Metalloxid oder Habmetalloxid aufweist, ist aus der Praxis bekannt. Auf dem Metalloxid oder Habmetalloxid ist eine selbst organisierende Schicht (SAM) mit bifünktionellen Molekülen, so genannten "Linkern" angeordnet. Die Linker sind mit einer ersten funktionellen Gruppe kovalent an die Oberfläche des Trägers und mit einer zweiten funktionellen Gruppe kovalent an ein Rezeptormolekül gebunden, das für einen zu detektierenden Liganden bindungsspezifisch ist. Derartige Sensor-Chips werden zum Nachweis und/oder zur Messung der Konzentration des Liganden in einer zu untersuchenden Lösung verwendet. Dabei wird die Lösung, für eine vorgegebene Zeitdauer mit der Oberfläche in Kontakt gebracht, um den Liganden die Möglichkeit zu geben, an die Rezeptoren zu binden. Danach wird der Träger gespült, um die Lösung und eventuell darin noch enthaltene freie Liganden von der Oberfläche zu entfernen. An der Oberfläche bleiben dann nur die Rezeptoren und die daran gebundenen Liganden zurück. In Abhängigkeit von der Bindung der Liganden an die Rezeptoren wird eine Lumineszenzstrahlung erzeugt. Diese wird mit Hilfe eines optischen Sensors gemessen. Aus der Amplitude des Messsignals lässt sich mit Hilfe einer zuvor aufgezeichneten Kallbraflonskurve die Konzentration des Liganden in der Lösung bestimmen. Die Kollbrationskurve wird mit Hilfe von Referenzanalyfen gemessen, die den Liganden in bekannter Konzentration enthalten. Der Sensorchip hat den Nachteil, dass die Konzentration des Liganden nur in einem relativ eng begrenzten Konzentrationsbereich mit hoher Empfindlichkeit gemessen werden kann. Ist die Konzentration des Liganden höher als dieser Konzentrationsbereich, gerät das Messsignal in die Begrenzung. Ist die Konzentration niedriger als der Konzentrationsbereich, ist das Messsignal zu schwach und verrauscht.

Aus WO 00/43539 A2 ist auch bereits ein Sensor-Chip bekannt, der auf einem Träger ein Polymer-Netzwerk aufweist, das kovalent an der Oberfläche des Trägers fixiert ist. Auf und/oder in dem Polymer-Netzwerk sind Rezeptoren angeordnet, die kovalent an das Polymer-Netzwerk gebunden sind. Im Vergleich zu einem Sensor-Chip, bei dem die Rezeptoren in einer Monolage auf dem Träger angeordnet sind, lässt sich mit Hilfe eines Polymer-Netzwerks eine höhere Rezeptor-Dichte erreichen. Zum Nachweis und/oder zur Messung der Konzentration des Liganden in einer zu untersuchenden Lösung wird die Lösung für eine vorgegebene Zeitdauer mit dem Polymemetzwerk in Kontakt gebracht. Danach wird der Träger mit dem Polymer-netzwerk gespült, um freie Liganden aus dem Polymernetzwerk zu entfernen. Der Nachweis des Bindungsereignisses erfolgt auch hier durch Messung von Lumineszenzstrahlung, die in Abhängigkeit von der Bindung des Liganden an den Rezeptor erzeugt und mit Hilfe einer CCD-Kamera detektiert wird. Auch bei diesem Sensor-Chip muss die Konzentration des Liganden in einem relativ eng begrenzten Konzentrationsbereich liegen, damit eine ausreichende Messgenauigkeit erreicht wird. In der Praxis bedeutet dies, dass die zu untersuchende Lösung verdünnt und/oder aufkonzentriert werden muss, wenn die Ligandenkonzentration größer oder geringer ist als der Messbereich des Sensor-Chips.

Aus EP 1 496 363 A1 ist ferner ein Biosensor mit einer SPR-Chipoberfläche bekannt, an der partikelförmige Komponenten der Formel (X-W²-PEG-W¹-L)ₓ-PCL-(L-W¹-PEG-W²-Y)_{y} über die Gruppe x gebunden vorliegen, wobei PCL ein Metallpartikel, ein Metalloxid oder ein Halbleiterparfikel, X eine funktionelle Gruppe, Y eine weitere Gruppe und PEG Polyethylenglykol (-CH₂CH₂-)ₙ ist, also eine lineare Kette. Nach Angabe der Offenlegungsschrift soll durch die partikelförmigen Komponenten eine hohe Sensitivität des Biosensors erreicht werden. Der Biosensor ermöglicht jedoch nur eine relativ geringe Messdynamik.

Es besteht deshalb die Aufgabe, einen Sensor-Chip und ein Verfahren der eingangs genannten Art zu schaffen, die bei der Messung eine hohe Dynamik ermöglichen.

Diese Aufgabe wird bezüglich des Sensor-Chips dadurch gelöst, dass an der Oberfläche des Trägers zusätzlich zu der die ersten Rezeptoren aufweisenden ersten Teststelle mindestens eine zweite Teststelle vorgesehen ist, an der ein quellbares Polymer-Netzwerk fixiert ist, und dass auf und/oder in dem Polymer-Netzwerk zweite Rezeptoren immobilisiert sind, die für den zu detektierenden Liganden bindungsspezifisch sind.

Der Erfindung liegt die überraschende Erkenntnis zu Grunde, dass ein Sensor-Chip, bei dem die Rezeptoren in oder auf einem Polymer-Netzwerk immobilisiert sind, eine andere Messcharakteristik aufweist als ein Sensor-Chip, bei dem die Rezeptoren in einer Monolage und/oder in mehreren übereinander geschichteten Moleküllagen (Polylage) auf der Oberfläche immobilisiert sind, und dass durch die Kombination dieser beiden Immobilisierungsarten auf nur einem Träger die Konzentration des Liganden in einem großen Konzentrationsbereich mit hoher Messgenauigkeit bestimmt werden kann. Durch die Bindung der zweiten Rezeptoren an ein Polymemetzwerk verändert sich die Bindungsafinität zwischen den zweiten Rezeptoren und dem Liganden, wodurch sich die Sättigungskonzentration und die Konzentration, bei der die Hälfte der Rezeptoren innerhalb einer vorgegebenen Zeitdauer nach dem Inkontaktbringen einer zu untersuchenden, den Liganden enthaltenden Lösung, an einen Liganden gebunden sind, verändern. Der Sensor-Chip ermöglicht also eine größere Dynamik als ein Sensor-Chip, bei dem die Rezeptoren nur auf eine der beiden Arten immobilisiert sind. Bei geringen Ligandenkonzentrationen ergibt die erste, die Mono- oder Polylage aufweisende Teststelle ein höheres Signal als die zweite, mit dem Polymernetzwerk beschichtete Teststelle. Bei hohen Ligandenkonzentrationen ist es umgekehrt. Dort ergibt sich an der zweiten Teststelle das größere Messsignal und eine deutlich höhere Empfindlichkeit. Geringe Ligandenkonzentrationen lassen sich also gut mit der ersten Teststelle und hohe Ligandenkonzentrationen gut mit der zweiten Teststelle nachweisen. Die Rezeptoren können an die Oberfläche kovalent oder nicht kovalent gebunden sein. Z.B. binden Antikörper an eine Polystyrol-Oberfläche nicht kovalent Die Rezeptoren können Nukleinsäuren oder Derivate davon (DNA, RNA, PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen. Unter einem Sensor-Chip wird sowohl ein Chip verstanden, der als Träger einen Halbleiterchip aufweist, als auch ein Chip verstanden bei dem der Träger aus einem anderen Werkstoff als einem Halbleiterwerkstoff besteht.

Die vorstehend genannte Aufgabe wird bezüglich des Sensor-Chips auch dadurch gelöst, dass in die Oberfläche des Trägers mindestens ein Detektor zum Detektieren der Bindung der Liganden an erste Rezeptoren und/oder die zweiten Rezeptoren integriert ist, und dass das Polymer-Netzwerk direkt oder indirekt über mindestens eine Lage der ersten Rezeptoren an dem mindestens einen Detektor fixiert ist.

Die Konzentration des Liganden kann dann über einen weiten Konzentrationsbereich mit hoher Auflösung gemessen werden. Dabei ist es sogar möglich, in einem Konzentrationsbereich, der sich über mindestens zwei, insbesondere drei und bevorzugt vier Dekaden erstreckt, Messungen vorzunehmen.

Das Polymer-Netzwerk ist bevorzugt ein quellbares Polymer-Netzwerk. Das Polymer des Polymernetzwerks kann dann bei der Herstellung des Sensor-Chips in einem wasserbasierten Lösungsmittel gelöst auf die Trägeroberfläche gedruckt werden.

Bei einer vorteilhaften Ausführungsform der Erfindung weist das Polymer-Netzwerk hydrophile Copolymere auf Die Rezeptoren können dann in wässriger Lösung mit noch größerer Dichte an der zweiten Teststelle angeordnet sein.

Das Polymer-Netzwerk enthält bevorzugt ein wasserlösbares, präformiertes Polymer, das mindestens eine photovernetzbare Gruppe aufweist, insbesondere eine Benzophenon-Gruppe. Die Moleküle für das Polymer-Netzwerk können dann bei der Herstellung des Sensor-Chips auf einfache Weise in Lösung auf den Träger aufgebracht und danach durch Bestrahlung mit UV-Strahlung zu dem Polymer-Netzwerk vernetzt werden.

Vorteilhaft ist, wenn dass das Polymer-Netzwerk ein Polymer enthält, das eine Haftvermittlergruppe aufweist, insbesondere Phosphonsäure. Der Träger kann dann zumindest an seiner Oberfläche aus Glas oder Siliziumdioxid bestehen und das Polymer kann nicht kovalent an die Oberfläche gebunden sein.

Bei einer bevorzugten Ausgestaltung der Erfindung weisen die ersten Rezeptoren und die zweiten Rezeptoren die gleichen Moleküle auf. Der Sensor-Chip lässt sich dann besonders kostengünstig herstellen.

Bei einer anderen Ausführungsform der Erfindung weisen die ersten Rezeptoren und die zweiten Rezeptoren unterschiedliche Moleküle. Dabei sind diese Moleküle für denselben Liganden bindungsspezifisch.

Vorteilhaft ist, wenn die ersten Rezeptoren über eine Silangruppe an der Oberfläche immobilisiert sind. Die ersten Rezeptoren können dadurch mit hoher Dichte fest an der Oberfläche des Trägers verankert werden.

Beispielsweise ist die Oberfläche des Trägers durchgängig über die erste Teststelle und die zweite Teststelle mit den in der Monolage und/oder den in mehreren übereinander geschichteten Moleküllagen angeordneten ersten Rezeptoren beschichtet, wobei das Polymer-Netzwerk auf den ersten Rezeptoren angeordnet und indirekt über diese und/oder zwischen diesen an der Oberfläche des Trägers kovalent fixiert ist, Der Sensor-Chip lässt sich dann kostengünstig herstellen, wobei zunächst ganzflächig die Monolage, z.B. durch Eintauchen des Trägers In eine Rezeptor-Lösung und anschließendes Herausziehen des Trägers aus der Rezeptor-Lösung, und danach das die Rezeptoren enthaltende Polymer-Netzwerk auf den Träger aufgebracht wird,

Bei einer vorteilhaften Ausgestaltung der Erfindung sind an den Teststellen in die Oberfläche des Trägers Detektoren zum Detektieren der Bindung der Liganden an die Rezeptoren angeordnet sind, die mit dem Träger eine Einheit bilden. Dabei ist es sogar möglich, dass in den Träger eine Auswerteeinrichtung integriert ist, die mit Messsignalausgängen der Detektoren verbunden ist. Mit Hilfe der Auswerteeinrichtung können die Messsignale der Detektoren mit vorgegebenen Aussteuerungsbereichen verglichen werden, um ein Messsignal auszuwählen, das in einem günstigen Aussteuerungsbereich liegt. Dieses Messsignal kann dann an einen Messsignalausgang weitergeleitet und oder zur Bestimmung der Ligandenkonzentration weiterverarbeitet werden. Mit Hilfe mindestens eines weiteren Messsignals kann ggf. eine Plausibilitätskontrolle durchgeführt werden,

Bei einer bevorzugten Ausführungsform der Erfindung weist der Träger einen Halbleiterwerkstoff auf, wobei der wenigstens eine Detektor in den Halbleiterwerkstoff integriert ist, und wobei das die Rezeptoren enthaltene Polymer-Netzwerk vorzugsweise direkt auf der Oberfläche des Detektors angeordnet ist. Dadurch ergibt sich eine hohe Detektionsempfindlichkeit. Dabei kann der wenigstens eine Detektor kann eine Photodiode sein.

Bei einer bevorzugten Ausführungsförm der Erfindung sind die Detektoren optische Detektoren zum Erfassen einer in Abhängigkeit von der Bindung der Liganden an die Rezeptoren auftretenden Lumineszenzstrahlung. Die Lumineszenzstrahlung kann dann direkt an den Teststellen und somit mit großer Detektionsempfindlichkeit gemessen werden, Die Lumineszenzstrahlung kann durch Bestrahlung der Teststellen mit einer Anregungsstrahlung induziert, die eine von der Lumineszenzstrahlung abweichende Wellenlänge aufweist Die Lumineszenzstrahlung kann aber auch durch eine chemische Reaktion erzeugt werden (Chemilumineszenz).

Bei einer anderen vorteilhaften Ausführungsform der Erfindung sind die Detektoren zur Messung eines in Abhängigkeit von der Bindung der Liganden an die Rezeptoren auftretenden elektrischen Felds und/oder einer elektrischen Eigenschaft der Liganden ausgebildet. Zur Messung des elektrischen Felds ist als Detektor vorzugsweise ein ISFET vorgesehen. Zur Messung der elektrischen Eigenschaft der Rezepotor-Liganden-Komplexe kann ein impedanzsensor vorgesehen sein. Die Bindung des Liganden an die Rezeptoren kann also auch markierungsfrei detektiert werden.

Vorteilhaft ist, wenn auf dem Träger mehrere der Sensor-Anordnungen, jeweils bestehend aus den an der ersten Teststelle immobilisierten ersten Rezeptoren, dem an der zweiten Teststelle angeordneten quellbaren Polymer-Netzwerk und den daran immobilisierten zweiten Rezeptoren vorgesehen sind, und wenn die Rezeptoren von mindestens zwei dieser Anordnungen für unterschiedliche Liganden bindungsspezifisch sind und/oder eine unterschiedliche Oberflächenbelegungsdichte aufweisen. Mit Hilfe des Sensor-Chips kann dann die Konzentration mehrerer in einer zu untersuchenden Lösung enthaltenden Liganden gleichzeitig gemessen werden. Wenn die Rezeptoren der einzelnen Sensor-Anordnungen unterschiedlichen Oberflächenbelegungsdichten aufweisen, kann die Konzentration der Liganden mit Hilfe des Sensor-Chips in einem noch größeren Konzentrationsbereich gemessen werden. Die Sensor-Anordnungen sind bevorzugt matrixförmig auf dem Träger angeordnet und voneinander beabstandet.

Bei einer vorteilhaften Ausgestaltung der Erfindung ist der Träger ein flexibles Band, Insbesondere eine Polymerfolie, wobei die Sensor-Anordnungen in Erstreckungsrichtung des Bands versetzt zueinander angeordnet sind. Der Sensor-Chip kann dann eine Vielzahl von Sensor-Anordnungen aufweisen und lässt sich dennoch platzsparend lagern, beispielsweise wenn das Band zu einer Rolle aufgewickelt ist. An dem Träger können maschinenlesbare Markierungen vorgesehen sein, in denen Informationen über die den Markierungen jeweils zugeordneten Sensor-Anordnungen, insbesondere über die Art und/oder die Dichte der an den Teststellen angeordneten Rezeptoren gespeichert sind. Das Band kann eine Perforierung für den Eingriff einer Transport- und/oder Positionierungseinrichtung aufweisen.

Der Träger besteht vorzugsweise aus Kunststoff, Insbesondere aus einem transparenten Kunststoff, wie z.B. PMMA Die Teststellen können dann durch den Träger hindurch optisch untersucht werden.

Vorteilhaft ist, wenn der Sensor-Chip eine Begrenzungswand einer Messkammer bildet, und wenn die Messkammer vorzugsweise als Durchflussmesskammer mit mindestens einer Einlassöffnung und wenigstens einer Auslassöffnung ausgebildet ist. Ein zu untersuchender flüssiger oder fließfähiger Analyt und/oder eine Spülflüssigkeit können dann auf einfache Weise beispielsweise mittels einer Pumpe über die Einlassöffnung in die Messkammer geleitet und dort mit den Teststellen in Kontakt gebracht werden.

Bei einer bevorzugten Ausgestaltung der Erfindung weist die Messkammer mindestens zwei Kompartimente auf, an die jeweils mindestens eine an der Oberfläche des Trägers angeordnete Sensor-Anordnung angrenzt. Mindestens eines der Kompartimente kann dann zum Kalibrieren des Sensor-Chips mit einem Referenzanalyten befüllt werden, der den zu untersuchenden Liganden in bekannter Konzentration enthält. In mindestens ein weiteres Kompartiment kann der zu untersuchende Analyt eingefüllt werden. Die einzelnen Kompartimente weisen bevorzugt jeweils eigene Einlass- und Auslassöfinungen auf.

Der erfindungsgemäße Sensorchip kann bei einem Verfahren zur Messung einer ersten Konzentration eines in einem zu untersuchenden Analyten enthaltenen ersten Liganden und einer größeren zweiten Konzentration eines in dem Analyten enthaltenen zweiten Liganden verwendet werden. Dabei werden zum Bestimmen der ersten Konzentration die Häufigkeit der Bindungen der ersten Liganden an die ersten Rezeptoren und zum Bestimmen der zweiten Konzentration die Häufigkeit der Bindungen der zweiten Liganden an die zweiten Rezeptoren gemessen. Aus den so erhaltenen Messwerten, den Oberflächenbelegungsdlchten des Trägers mit den ersten und zweiten Rezeptoren und bekannten Bindungskonstanten können dann mit Hilfe des Massenwirkungsgesetzes die Konzentrationen der Liganden berechnet werden.

Nachfolgend sind Ausftihrungsbeispiele der Erfindung anhand der Zeichnung näher erläutert Es zeigen zum Teil stärker schematisiert:
- Fig. 1: eine Aufsicht auf einen biologischen Sensor-Chip, der einen Träger hat, in den optische Detektoren integriert sind,
- Fig. 2: einen Teilquerschnitt durch ein erstes Ausführungsbeispiel des Sensor-Chips, wobei die auf dem Sensor-Chip immobilisierten Rezeptoren schematisch dargestellt sind,
- Fig. 3: eine Darstellung ähnlich Fig. 2, wobei Jedoch an einige Rezeptoren Nachweisantikörper gebunden sind, an die ein Marker gekoppelt ist,
- Fig. 4: eine graphische Darstellung zweier mit Hilfe der in den Träger integrierten Detektoren aufgezeichneter Messsignale, wobei auf der Abszisse der dekadische Logarithmus der Ligandenkonzentration in g/ml und auf der Ordinate die Amplitude des Messsignals in einer willkürlich gewählten Einheit aufgetragen sind,
- Fig. 5: Höhenprofile von in Monolage auf einer Trügeroberflöche angeordneten Rezeptoren und von an der Trägeroberfläche fixierten Polymernetzwerken, in denen Rezeptoren immobilisiert sind, wobei auf der Abszisse die Position und auf der Ordinate die Höhe aufgetragen ist,
- Fig.6: einen Teilquerschnitt durch ein zweites Ausführungsbeispiel des Sensor-Chips, und

- Fig. 7: einen Querschnitt durch eine Durchflussmesskammer.

Ein Im Ganzen mit 1 bezeichneter Sensor-Chip weist einen Träger 2 auf, an dessen Oberfläche 3 mehrere Teststellen 4a, 4b angeordnet sind. An den einzelnen Teststellen 4a, 4b Ist Jeweils ein optischer Detektor 5a, 5b, wie z.B. eine Fotodiode, oberflächennah In den Träger 2 integriert.

Die Teststellen 4a, 4b sind in an sich bekannter Weise mit einem in der Zeichnung nicht näher dargestellten bifunktionellen Silan beschichtet, das mit einer ersten funktionellen Gruppe kovalent an die Oberfläche bindet. Das Silan ist bevorzugt als Polylage auf die Oberfläche 3 aufgebracht, beispielsweise mit Hilfe des in EP 1 176 422 B1 beschriebenen Verfahrens. Das Silan kann aber auch in einer selbst organisierenden Monolage (SAM) an der Oberfläche 3 vorliegen.

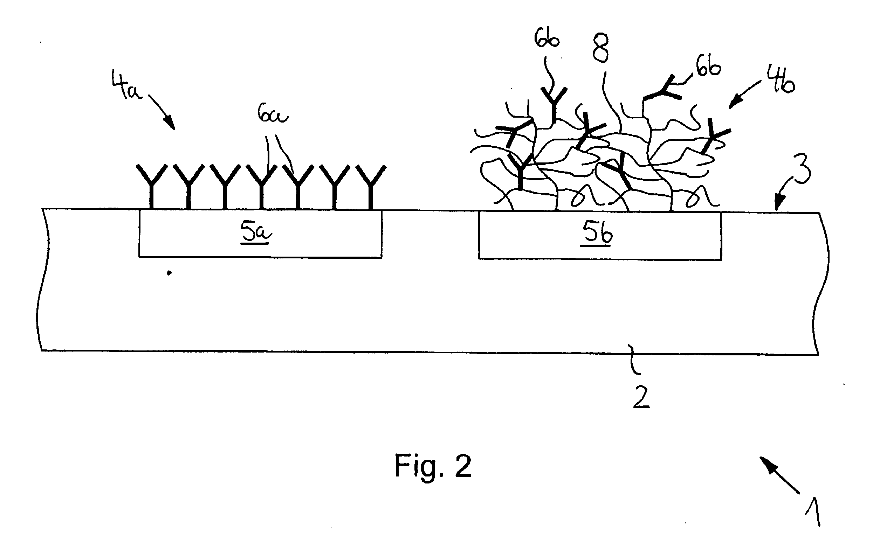

An ersten Teststellen 4a sind auf dem Silan erste Rezeptoren 6a in einer Monolage immobilisiert (Fig. 2). Die ersten Rezeptoren 6a, die beispielsweise Antikörper sein können, sind für einen zu detektierenden Liganden 7 bindungsspezifisch. Die ersten Rezeptoren 6a sind jeweils an eine zweite funktionelle Gruppe des Silans kovalent gebunden. Sie sind direkt über den optischen Detektoren 5a angeordnet.

An zweiten Teststellen 4b ist auf dem Silan eine quellbares Polymer-Netzwerk 8 angeordnet, das miteinander vemetzte, lineare Polymermoleküle aufweist, die in der Zeichnung nur schematisch als Linien dargestellt sind. Die Polymermoleküle sind kovalent an die zweiten funktionellen Gruppen des Silans gebunden und somit auf der Oberfläche 3 immobilisiert.

Wie in Fig. 2 erkennbar ist, sind auf und/oder in dem Polymer-Netzwerk 8 zweite Rezeptoren 6b immobilisiert, die für denselben Liganden 7 bindungsspezifisch sind wie die ersten Rezeptoren 6a. Die ersten Rezeptoren 6a und die zweiten Rezeptoren 6b weisen zu diesem Zweck gleiche Moleküle auf. Die zweiten Rezeptoren 6b sind jeweils kovalent an ein lineares Polymermolekül des Polymer-Netzwerks 8 gebunden. Sie sind direkt über den optischen Detektoren 5b angeordnet.

Zur Messung der Konzentration der Liganden 7, von denen vermutet wird, dass sie In einer zu untersuchenden Lösung enthalten sind, wird die Lösung beispielsweise mittels einer Pipette oder eines Strahldruckers derart auf die Teststellen 4a, 4b aufgetragen, dass sie mit den Rezeptoren 6a, 6b in Kontakt gerät. Dann wird eine vorgegebene erste Zeitdauer abgewartet, um den Liganden 7 die Möglichkeit zu geben, zu den Rezeptoren 6a, 6b zu diffundieren und an diese zu binden. Danach wird die Oberfläche 3 des Trägers 2 mit einer Spülflüssigkeit gespült, um nicht an einen Rezeptor 6a, 6b gebundene Bestandteile der Lösung von der Oberfläche 2 zu entfemen.

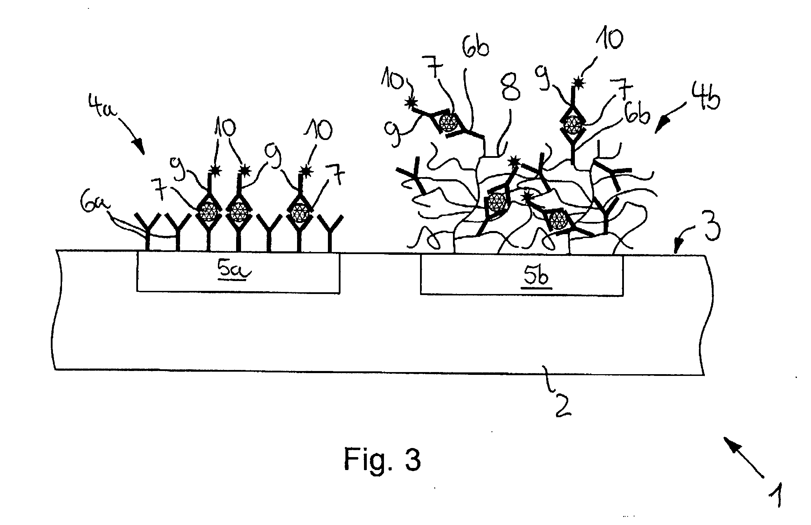

In einem weiteren Schritt wird eine Lösung auf die Oberfläche 2 aufgebracht, die einen Nachweisantikörper 9 enthält, der mit einem Marker 10 markiert ist, der durch Bestrahlung mit einer Anregungsstrahlung zur Abgabe einer Lumineszenzstrahlung anregbar ist. Der Nachweisantikörper 9 bindet an die Liganden 7, nicht Jedoch an freie Rezeptoren 6a, 6b. Nachdem die Lösung mit den Nachweisantikörpem für eine vorgegebene zweite Zeitdauer mit den Teststellen 4a, 4b In Kontakt gebracht wurde, wird die Oberfläche 3 erneut mit einer Spülflüssigkeit gespült, um eventuell noch vorhandene ungebundene Nachweisantikörper 9 von der Oberfläche 2 zu entfernen (Fig. 3). Danach wird die Oberfläche 2 mit der Anregungsstrahlung bestrahlt und die in Abhängigkeit von der Bindung der Liganden 7 an die Rezeptoren 6a, 6b auftretende Lumineszenzstrahlung wird mit Hilfe der Detektoren 5a, 5b jeweils für die einzelnen Teststellen 4a, 4b gemessen.

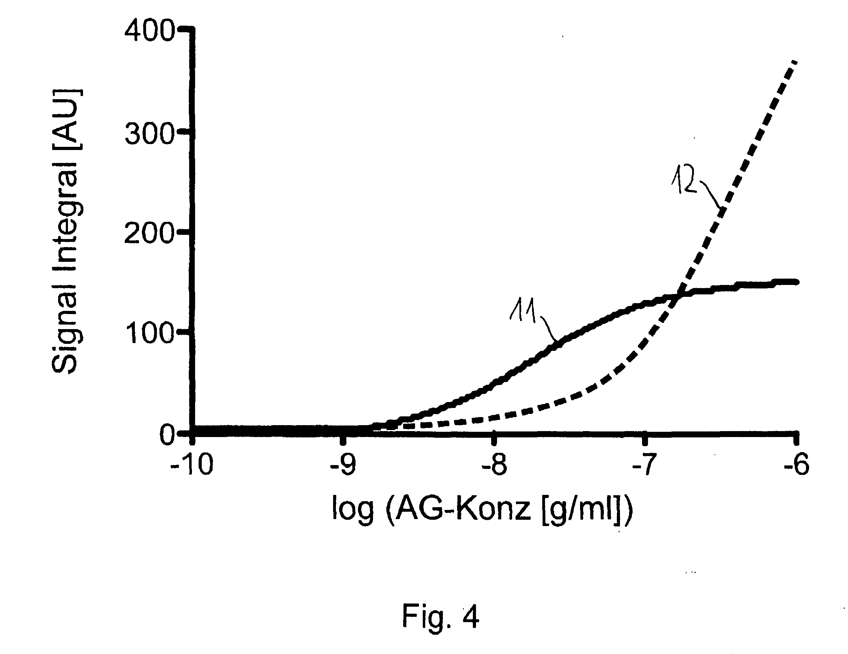

In Fig. 4 sind die Messsignale der einzelnen Detektoren 5a, 5b in Abhängigkeit von der Konzentration der Liganden 7 graphisch dargestellt. Die durchgezogene Kurve 1 zeigt die Charakteristik der an den ersten Rezeptoren 6a (Monologe) gemessenen Detektorsignale. Die strichlinierte Kurve 12 gibt die Messsignalcharakteristik für die an den zweiten Rezeptoren 6b (in und/oder auf dem Polymer-Netzwerk) gemessenen Detektorsignale wieder. Deutlich ist erkennbar, dass die ersten Detektoren 5a bei geringen Ligandenkonzentrationen ein größeres Messsignal aufweisen als die zweiten Detektoren 5b. Bei hohen Konzentrationen geraten die Messsignale der ersten Detektoren 5a in die Begrenzung. Die Messsignale der zweiten Detektoren 5b weisenjedoch bei diesen Konzentrationen noch eine relativ hohe Dynamik auf Durch die Kombination der in Monolage immobilisierten ersten Rezeptoren 6a mit den in und/oder auf dem Polymer-Netzwerk 8 immobilisierten zweiten Rezeptoren 6b wird also die Dynamik der Messung stark vergrößert, und zwar in dem in Fig. 4 gezeigten Beispiel um mindestens eine Dekade.

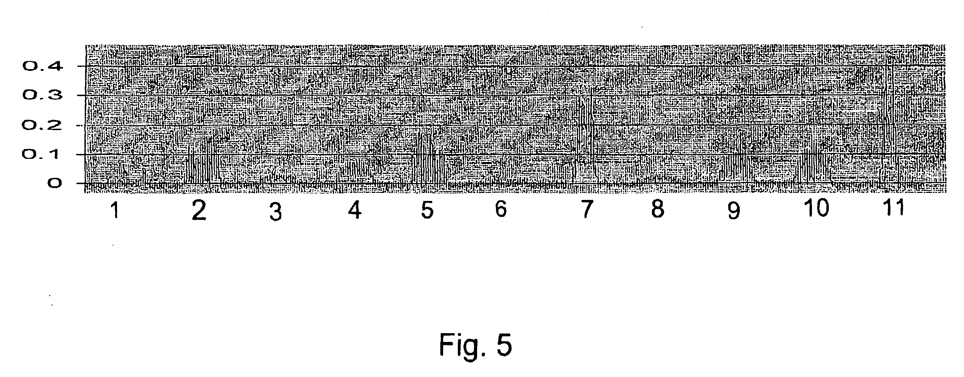

In Fig. 5 sind Höhenprofile von in Monolage (Positionen 1 und 6) auf einer Trägeroberfläche 3 angeordneten Rezeptoren 6a und von an der Trägeroberfläche 3 fixierten Polymernetzwerken 8, auf und/oder in denen Rezeptoren 6b angeordnet sind (Positionen 2 bis 5 und 7 bis 11) für unterschiedliche Rezeptorarten jeweils graphisch dargestellt. Deutlich ist erkennbar, dass die Polymernetzwerke eine größere Höhe und somit ein größeres Volumen aufweisen als die Monolagen. Somit kann bei Bindung der Rezeptoren 6b an ein Polymernetzwerk 8 eine größere Rezeptordichte pro Trägeroberfläche erreicht werden als bei in Monolage angeordneten Rezeptoren 6a.

Bei einem weiteren Ausführungsbeispiel wird die Oberfläche des Trägers 2 z.B. durch Dipcoating mit einer Silanschicht bedeckt Danach wird der Träger in einem Toluolbad mittels Ultraschallwellen gereinigt Dann werden eine erste Lösung aus ersten Rezeptoren 6a sowie einem Phosphatpuffer und eine zweite Lösung aus zweiten Rezeptoren 6b, dem Phosphatpuffer und einem in Wasser gelöstem, photovernetzbarem Polymer hergestellt Als erste Rezeptoren 6a und zweite Rezeptoren 6b werden die gleichen CRP-Antikörper verwendet. Die Konzentrationen in den beiden Lösungen werden folgendermaßen gewählt
- CRP-Antikörper: 250 µg/ml
- Photovemetzbares Polymer. 1 mg/ml
- Puffer. 10mM Phosphatpuffer

Danach wird der Träger 2 an den ersten Teststellen 4a mit Tropfen der ersten Lösung und an den zweiten Teststellen 4b mit Tropfen der zweiten Lösung beschichtet, beispielsweise mit dem Print-Roboter Sciflexarrayer S5 der Fa. Scienion. Die abgesetzten Volumina betragen für beide Lösungen etwa 1,6nl.

Für eine kovalente Anbindung der ersten Rezeptoren 6a in Monolage auf die Silanschicht wird der beschichtete Träger 2 bei einer Temperatur von etwa 4 C gelagert. Für die Anhaftung des durch eingebaute Benzophenongruppen photovemetzbaren Polymers auf der Träger-Oberfläche 3 und die kovalente Anbindung der Antikörper in das Polymer wird dieses mit Licht der Wellenlänge 365nm bestrahlt.

Nicht immobilisierte Residuen der gedruckten Lösungen werden durch Waschen des Trägers 2 in einem Waschpuffer entfernt Dieser gewährleistet gleichzeitig den Erhalt der Funktionsfahigkeit der Antikörper. Als Waschpuffer kann SSC-SDS-Puffer mit zugesetztem Tensid Tween20 zum Waschen der Träger verwendet werden. Anschließend wird der Träger 2 kurz mit deionisiertem Wasser abgespült.

Danach werden auf den Träger 40µl einer auf CRP zu untersuchenden Analytlösung pipettiert, nämlich eines Serums oder Plasmas oder einer Verdünnung eines Serums in einem Puffer. Die Analytlösung wird etwa eine Stunde lang auf dem Träger 2 belassen, um eine ausreichende Zahl von Rezeptor-Liganden-Komplexen zu erhalten. Danach wird der Träger 2 erneut mit dem Waschpuffer gewaschen.

Dann wird der Träger mit 40µl einer Lösung von CRP-speziflschen Nachweisantikörper 9 in 1OmM Phosphatpuffer pipettiert. An die Nachweisantikörper 9 werden zuvor kovalent fluorochrome Farbstoff-Moleküle (z.B. CyDye-NHS) gebunden. Die Nachweis-Antikörper-Lösung wird etwa eine Stunde lang auf dem Träger 2 inkubiert. Anschließend wird der Träger 2 erneut mit dem Waschpuffer gewaschen, um ihn danach mit Stickstofftrocken zu blasen.

Der so erhaltene Sensor-Chip 1 wird mit dem Fluoreszenzmessgerät Biodetect gemessen. Dabei werden die an die Nachweisantikörper 9 angebundenen Farbstoffmoleküle mit Licht der Wellenlänge 650nm angeregt. Das hierbei vom Farbstoff emittierte Licht der Wellenlänge 675nm wird mittels eine CCD-Kamera hinter einer monochromatischen Filtereinheit detektiert. Die aufgenommene Lichtintensität ist proportional der Menge der Farbstoffmoleküle und somit auch proportional der Zahl der Nachweis-Antikörper 9. Die Zahl der gemessenen Nachweis-Antikörper 9 ist ihrerseits ein Maß für die Menge der auf der Rezeptorschicht des Trägers gebundenen Analytmoleküle, Es kann somit die Menge der Analytmoleküle in der untersuchten Lösung (z.B. Serum von humanem Blut) bestimmt werden.

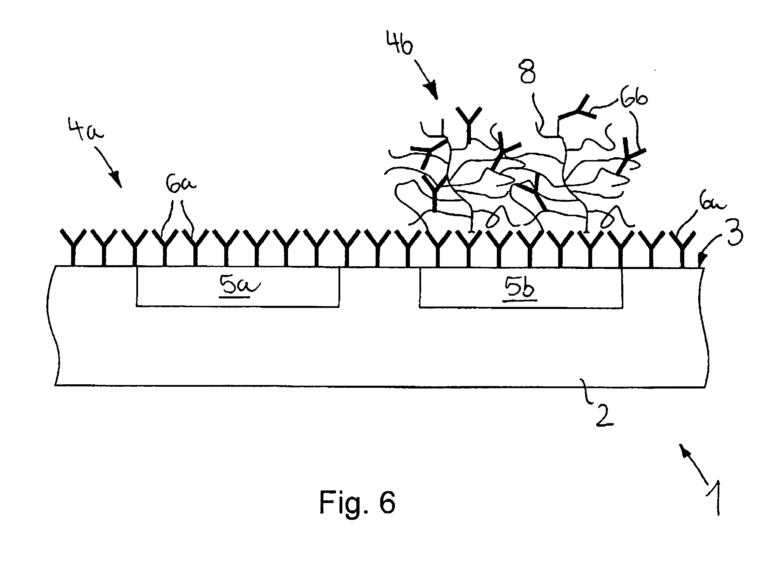

Bei dem in Fig. 6 gezeigten Ausführungsbeispiel ist die Oberfläche 4 des Trägers 2 durchgängig sowohl an den ersten Teststellen 4a als auch an den zweiten Teststellen 4b mit den in Monolage angeordneten ersten Rezeptoren 6a beschichtet An den zweiten Teststellen 4b ist auf den ersten Rezeptoren 6a das Polymer-Netzwerk 8 mit den zweiten Rezeptoren 6b angeordnet Dabei können die linearen Moleküle des Polymer-Netzwerks 8 an den ersten Rezeptoren 6a und/oder der Oberfläche 3 bzw. einer darauf befindlichen Haftvermittlerschicht, wie z.B. einer Silan-Schicht, fixiert sein.

Bei dem Ausführungsbeispiel gemäß Fig. 2 endet dagegen die die ersten Rezeptoren 6a aufweisende Oberfiächenbeschichtung mit Abstand zu den zweiten Teststellen 4b. Die linearen Moleküle des Polymer-Netzwerks 8 sind direkt an der Oberfläche 3 und/oder einer darauf befindlichen Haftvermütlerschicht fixiert.

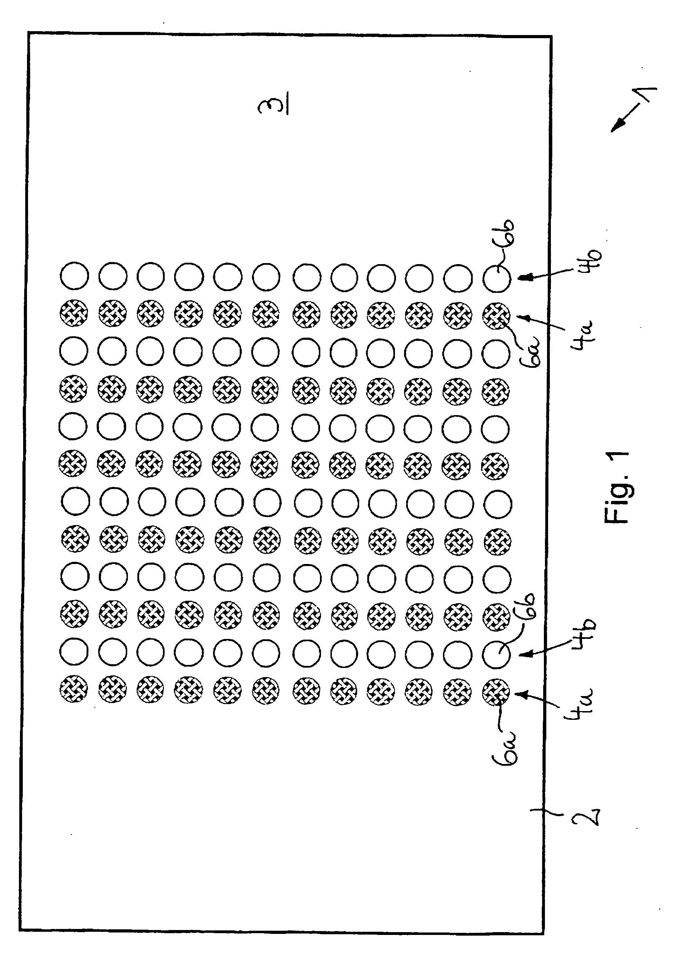

In Fig. 1 ist erkennbar, dass auf dem Träger 2 mehrere der Sensor-Anordnungen, jeweils bestehend aus den an der ersten Teststelle 4a immobilisierten ersten Rezeptoren 6a, dem an der zweiten Teststelle 4b angeordneten quellbaren Polymer-Netzwerk 8 und den daran immobilisierten zweiten Rezeptoren 6b vorgesehen sind. Dabei sind die Sensor-Anordnungen matrixförmig in mehreren Reihen und Spalten angeordnet und seitlich voneinander beabstandet. Die Rezeptoren 6a, 6b von Sensor-Anordnungen, die in derselben Zelle der Matrix angeordnet sind, weisen bezogen auf die Oberfläche 3 des Trägers 2 eine unterschiedliche Flächenbelegungsdichte auf, wodurch sich die Dynamik des Sensor-Chips 1 vergrö-ßert. Die Rezeptoren 6a, 6b von Sensor-Anordnungen, die in derselben Spalte der Matrix angeordnet sind, sind für unterschiedliche Liganden 7 bindungsspeziflsch. Somit kann mit nur einem Versuch die Konzentration mehrerer unterschiedlicher Liganden 7 in der zu untersuchenden Lösung bestimmt werden.

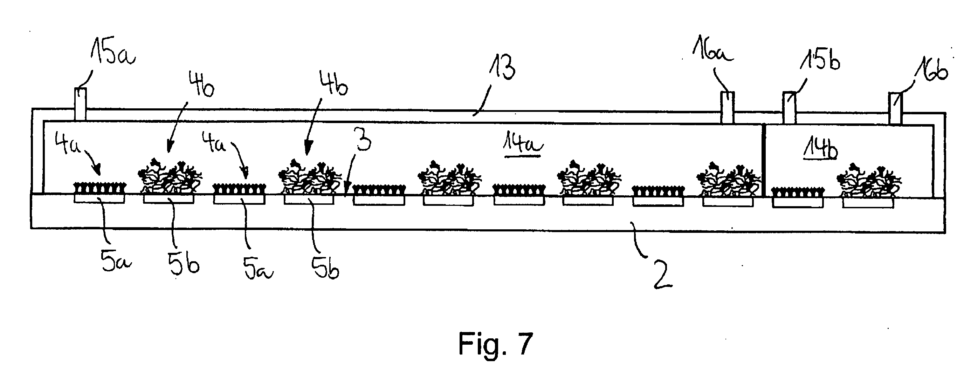

Bei dem in Fig. 7 gezeigten Ausführungsbeispiel bildet der Sensor-Chip 1 den Boden einer Durchflussmesskammer 13. Diese hat eine Innenhöhlung, die in zwei Kompartimente 14a, 14b unterteilt ist. Jedes Kompartiment 14a, 14b hat jeweils eine Einlassöffnung 15a, 15b und eine Auslassöffnung 16a, 16b. Das Kompartiment 14b kann zum Kalibrieren des Sensor-Chips 1 mit einem Referenzanalyten befüllt werden, der die Liganden 7 in bekannter Konzentration enthält. In das Kompartiment 14a kann ein zu untersuchender Analyt eingefüllt werden.

## Patentansprüche

1. Sensor-Chip (1), mit einem Träger (2), an dessen Oberfläche (3) Teststellen (4a, 4b) vorgesehen sind, an denen Rezeptoren (6a, 6b) immobilisiert sind, die für einen zu detektierenden Liganden (7) bindungsspezifisch sind, wobei die Rezeptoren (6a) in einer Monologe und/oder in mehreren übereinander geschichteten Moleküllagen an die Oberfläche (3) gebunden sind, **dadurch gekennzeichnet, dass** an der Oberfläche (3) des Trägers (2) zusätzlich zu der die ersten Rezeptoren (6a) aufweisenden ersten Teststelle (4a) oder auf dieser mindestens eine zweite Teststelle (4b) vorgesehen ist, an der ein Polymer-Netzwerk (8) fixiert ist, und dass auf und/oder in dem Polymer-Netzwerk (8) zweite Rezeptoren (6b) immobilisiert sind, die für den zu detektierenden Liganden (7) bindungsspezifisch sind.

2. Sensor-Chip (1), mit einem Träger (2), an dessen Oberfläche (3) mindestens eine Teststelle (4b) vorgesehen ist, an der ein Polymer-Netzwerk (8) fixiert ist, auf und/oder in dem Rezeptoren (6b) immobilisiert sind, die für einen zu detektierenden Liganden (7) bindungsspezifisch sind, **dadurch gekennzeichnet, dass** in die Oberfläche des Trägers (2) mindestens ein Detektor (5b) zum Detektieren der Bindung der Liganden (7) an erste Rezeptoren (6a) und/oder die zweiten Rezeptoren (6b) integriert ist, und dass das Polymer-Netzwerk (8) direkt oder indirekt über mindestens eine Lage der ersten Rezeptoren (6a) an dem mindestens einen Detektor (5b) fixiert ist.

3. Sensor-Chip (1) nach Anspruch 1 oder 2, **dadurch gekennzeichnet, dass** das Polymer-Netzwerk (8) ein quellbares Polymer-Netzwerk (8) ist.

4. Sensor-Chip (1) nach einem der Ansprüche 1 bis 3, **dadurch gekennzeichnet, dass** das Polymer-Netzwerk (8) hydrophile Copolymere aufweist.

5. Sensor-Chip nach einem der Ansprüche 1 bis 4, **dadurch gekennzeichnet, dass** das Polymer-Netzwerk (8) ein wasserlösbares, präformiertes Polymer enthält, das mindestens eine photovernetzbare Gruppe aufweist, insbesondere eine Benzophenon-Gruppe.

6. Sensor-Chip (1) nach einem der Ansprüche 1 bis 5, **dadurch gekennzeichnet, dass** das Polymer-Netzwerk (8) ein Polymer enthält, das eine Haftvermittlergruppe aufweist, Insbesondere Phosphonsäure.

7. Sensor-Chip (1) nach einem der Ansprüche 1 bis 6, **dadurch gekennzeichnet, dass** die ersten Rezeptoren (6a) und die zweiten Rezeptoren (6b) die gleichen Moleküle aufweisen.

8. Sensor-Chip (1) nach einem der Ansprüche 1 bis 7, **dadurch gekennzeichnet, dass** die ersten Rezeptoren (6a) und die zweiten Rezeptoren (6b) unterschiedliche Moleküle aufweisen.

9. Sensor-Chip (1) nach einem der Ansprüche 1 bis 8, **dadurch gekennzeichnet, dass** die ersten Rezeptoren (6a) über eine Silangruppe an der Oberfläche immobilisiert sind.

10. Sensor-Chip (1) nach einem der Ansprüche 1 bis 9, **dadurch gekennzeichnet, dass** die Oberfläche (3) des Trägers (2) durchgängig über die erste Teststelle (4a) und die zweite Teststelle (4b) mit den in der Monolage und/oder den in mehreren übereinander geschichteten Moleküllagen angeordneten ersten Rezeptoren (6a) beschichtet ist, und dass das Polymer-Netzwerk (8) auf den ersten Rezeptoren (6a) angeordnet und indirekt über diese und/oder zwischen diesen an der Oberfläche (3) des Trägers (2) kovalent fixiert ist

11. Sensor-Chip (1) nach einem der Ansprüche 1 bis 10, **dadurch gekennzeichnet, dass** an den Teststellen in die Oberfläche (3) des Trägers (2) Detektoren (5a, 5b) zum Detektieren der Bindung der Liganden (7) an die Rezeptoren (6a, 6b) angeordnet sind, die mit dem Träger eine Einheit bilden.

12. Sensor-Chip (1) nach einem der Ansprüche 1 bis 11, **dadurch gekennzeichnet, dass** der Träger (2) einen Halbleiterwerkstoff aufweist und dass der wenigstens eine Detektor (5b) in den Halbleiterwerkstoff integriert ist, und dass das die Rezeptoren (6b) enthaltene Polymer-Netzwerk (8) vorzugsweise direkt auf der Oberfläche des Detektors (5b) angeordnet ist.

13. Sensor-Chip (1) nach einem der Ansprüche 1 bis 12, **dadurch gekennzeichnet, dass** die Detektoren (5a, 5b) optische Detektoren (5a, 5b) zum Erfassen einer in Abhängigkeit von der Bindung der Liganden (7) an die Rezeptoren (6a, 6b) auftretenden Lumineszenzstrahlung sind.

14. Sensor-Chip (1) nach einem der Ansprüche 1 bis 13, **dadurch gekennzeichnet, dass** die Detektoren (5a, 5b) zur Messung eines in Abhängigkeit von der Bindung der Liganden (7) an die Rezeptoren (6a, 6b) auftretenden elektrischen Felds und/oder einer elektrischen Eigenschaft der Liganden (7) ausgebildet sind.

15. Sensor-Chip (1) nach einem der Ansprüche 1 bis 14, **dadurch gekennzeichnet, dass** auf dem Träger (2) mehrere der Sensor-Anordnungen, jeweils bestehend aus den an der ersten Teststelle (4a) immobilisierten ersten Rezeptoren (6a), dem an der zweiten Teststelle (4b) angeordneten quellbaren Polymer-Netzwerk (8) und den daran immobilisierten zweiten Rezeptoren (6b) vorgesehen sind, und dass die Rezeptoren (6a, 6b) von mindestens zwei dieser Anordnungen für unterschiedliche Liganden (7) bindungsspezifisch sind und/oder eine unterschiedliche Oberflächenbelegungsdichte aufweisen.

16. Sensor-Chip (1) nach einem der Ansprüche 1 bis 15, **dadurch gekennzeichnet, dass** der Träger (2) ein flexibles Band ist, insbesondere eine Polymerfolie, und dass die Sensor-Anordnungen in Erstreckungsrichtung des Bands versetzt zueinander angeordnet sind.

17. Sensor-Chip (1) nach einem der Ansprüche 1 bis 16, **dadurch gekennzeichnet, dass** der Träger (2) aus Kunststoff besteht, vorzugsweise einem transparenten Kunststoff,

18. Sensor-Chip (1) nach einem der Ansprüche 1 bis 17, **dadurch gekennzeichnet, dass** er eine Begrenzungswand einer Messkammer bildet, und dass die Messkammer vorzugsweise als Durchflussmesskammer (1 3) mit mindestens einer Einlassöffnung (15a, 15b) und wenigstens einer Auslossöffnung (16a, 16b) ausgebildet ist.

19. Sensor-Chip (1) nach einem der Ansprüche 1 bis 18, **dadurch gekennzeichnet, dass** die Messkammer mindestens zwei Kompartimente (14a, 14b) aufweist, an die Jeweils mindestens eine an der Oberfläche (3) des Trägers (2) angeordnete Sensor-Anordnung angrenzt.

20. Verfahren zur Messung einer ersten Konzentration eines in einem zu untersuchenden Analyten enthaltenen ersten Liganden und einer größeren zweiten Konzentration eines in dem Analyten enthaltenen zweiten Liganden, **dadurch gekennzeichnet, dass** der Analyt mit den Teststellen eines Sensorchips nach einem der Ansprüche 1 bis 16 in Kontakt gebracht wird, und dass zum Bestimmen der ersten Konzentration die Häufigkeit der Bindungen der ersten Liganden an die ersten Rezeptoren (6a) und zum Bestimmen der zweiten Konzentration die Häufigkeit der Bindungen der zweiten Liganden an die zweiten Rezeptoren (6b) gemessen werden.

21. Verfahren nach Anspruch 20, **dadurch gekennzeichnet, dass** sich die erste Konzentration und die zweite Konzentration um mindestens eine Dekade voneinander unterscheiden.

22. Verfahren zur Messung der Konzentrationen von in zwei zu untersuchenden Analyten enthaltenen Liganden, wobei mindestens zwei baugleiche Sensor-Chips (1) nach Anspruch 2 bereitgestellt werden,
- wobei ein erster Analyt mit mindestens einer Teststelle eines ersten Sensorchips (1) derart In Kontakt gebracht wird, dass in dem ersten Analyt enthaltene erste Liganden (7) an die an der Teststelle befindlichen Rezeptoren (6a, 6b) binden, wobei ein Messwert für die Häufigkeit der Bindungen der ersten Liganden an die Rezeptoren erfasst und anhand des Messwerts die Konzentration der ersten Liganden bestimmt wird,
- wobei ein zweiter Analyt mit mindestens einer Teststelle eines zweiten Sensorchips (1) derart in Kontakt gebracht wird, dass in dem zweiten Analyt enthaltene zweite Liganden (7) an die an der Teststelle befindlichen Rezeptoren (6a, 6b) binden, wobei ein Messwert für die Häufigkeit der Bindungen der zweiten Liganden an die Rezeptoren erfasst und anhand des Messwerts die Konzentration der zweiten Liganden bestimmt wird, und
- wobei sich die Konzentrationen der ersten und zweiten Liganden (7) um mindestens zwei, insbesondere drei und bevorzugt 4 Dekaden voneinander unterscheiden.

## Claims

1. Sensor chip (1) with a support (2) on whose surface (3) test sites (4a, 4b) are provided for to which receptors (6a, 6b) are immobilized which bind specifically to a ligand (7) to be detected, wherein the receptors (6a) are bound to the surface (3) in a monolayer and/or in multiple molecular layers layered upon one another, **characterized in that**, in addition to the first test site (4a) having the first receptors (6a) or thereon, at least one second test site (4b) to which a polymeric network (8) is fixed is provided for on the surface (3) of the support (2), and that second receptors (6b) which bind specifically to the ligand (7) to be detected are immobilized on and/or in the polymeric network (8).

2. Sensor chip (1) with a support (2) on whose surface (3) at least one test site (4b) is provided for to which a polymeric network (8) is fixed on and/or in which receptors (6b) which bind specifically to a ligand (7) to be detected are immobilized, **characterized in that** at least one detector (5b) for detecting binding of the ligands (7) to first receptors (6a) and/or the second receptors (6b) is integrated in the surface of the support (2), and that the polymeric network (8) is fixed directly or indirectly via at least one layer of the first receptors (6a) to the at least one detector (5b).

3. Sensor chip (1) according to Claim 1 or 2, **characterized in that** the polymeric network (8) is a swellable polymeric network (8).

4. Sensor chip (1) according to any of Claims 1 to 3, **characterized in that** the polymeric network (8) has hydrophilic copolymers.

5. Sensor chip according to any of Claims 1 to 4, **characterized in that** the polymeric network (8) comprises a water-soluble, pre-formed polymer which has at least one photo-crosslinkable group, in particular a benzophenone group.

6. Sensor chip (1) according to any of Claims 1 to 5, **characterized in that** the polymeric network (8) comprises a polymer which has a bonding agent group, in particular phosphonic acid.

7. Sensor chip (1) according to any of Claims 1 to 6, **characterized in that** the first receptors (6a) and the second receptors (6b) have the same molecules.

8. Sensor chip (1) according to any of Claims 1 to 7, **characterized in that** the first receptors (6a) and the second receptors (6b) have different molecules.

9. Sensor chip (1) according to any of Claims 1 to 8, **characterized in that** the first receptors (6a) are immobilized to the surface via a silane group.

10. Sensor chip (1) according to any of Claims 1 to 9, **characterized in that** the surface (3) of the support (2) is coated throughout via the first test site (4a) and the second test site (4b) with the first receptors (6a) arranged in the monolayer and/or in multiple molecular layers layered upon one another, and that the polymeric network (8) is arranged on the first receptors (6a) and is covalently fixed to the surface (3) of the support (2) indirectly via and/or between these receptors.

11. Sensor chip (1) according to any of Claims 1 to 10, **characterized in that** detectors (5a, 5b) for detecting binding of the ligands (7) to the receptors (6a, 6b) are arranged at the test sites into the surface (3) of the support (2), which detectors form a unit with the support.

12. Sensor chip (1) according to any of Claims 1 to 11, **characterized in that** the support (2) has a semi-conductor material and that the at least one detector (5b) is integrated in the semi-conductor material and that the polymeric network (8) comprising the receptors (6b) is preferably arranged directly on the surface of the detector (5b).

13. Sensor chip (1) according to any of Claims 1 to 12, **characterized in that** the detectors (5a, 5b) are optical detectors (5a, 5b) for recording a luminescent radiation occurring as a function of binding of the ligands (7) to the receptors (6a, 6b).

14. Sensor chip (1) according to any of Claims 1 to 13, **characterized in that** the detectors (5a, 5b) are designed for measuring an electric field occurring as a function of binding of the ligands (7) to the receptors (6a, 6b) and/or an electric property of the ligands (7).

15. Sensor chip (1) according to any of Claims 1 to 14, **characterized in that** a plurality of the sensor arrangements, each consisting of the first receptors (6a) immobilized at the first test site (4a), the swellable polymeric network (8) arranged at the second test site (4b) and the second receptors (6b) immobilized thereto, are provided for on the support (2), and that the receptors (6a, 6b) of at least two of these arrangements bind specifically to different ligands (7) and/or have a different surface occupation density.

16. Sensor chip (1) according to any of Claims 1 to 15, **characterized in that** the support (2) is a flexible tape, in particular a polymeric film, and that the sensor arrangements are staggered in the direction of extent of the tape.

17. Sensor chip (1) according to any of Claims 1 to 16, **characterized in that** the support (2) consists of plastic, preferably a transparent plastic.

18. Sensor chip (1) according to any of Claims 1 to 17, **characterized in that** it forms a boundary wall of a measurement chamber and that the measurement chamber is preferably a measurement flow chamber (13) having at least one inlet opening (15a, 15b) and at least one outlet opening (16a, 16b).

19. Sensor chip (1) according to any of Claims 1 to 18, **characterized in that** the measurement chamber has at least two compartments (14a, 14b) which adjoin in each case at least one sensor arrangement arranged on the surface (3) of the support (2).

20. Process for measuring a first concentration of a first ligand present in an analyte to be studied and a larger second concentration of a second ligand present in the analyte, **characterized in that** the analyte is contacted with the test sites of a sensor chip according to any of Claims 1 to 16, and that the first concentration is determined by measuring the frequency of bindings of the first ligands to the first receptors (6a) and the second concentration is determined by measuring the frequency of bindings of the second ligands to the second receptors (6b).

21. Process according to Claim 20, **characterized in that** the first concentration and the second concentration differ from one another by at least one decade.

22. Process for measuring the concentrations of ligands present in two analytes to be studied, wherein at least two structurally identical sensor chips (1) according to Claim 2 are provided,
- wherein a first analyte is contacted with at least one test site of a first sensor chip (1) in such a way that first ligands (7) present in the first analyte bind to the receptors (6a, 6b) located at the test site, wherein a value of the frequency of bindings of the first ligands to the receptors is measured and the concentration of the first ligands is determined on the basis of the measured value,
- wherein a second analyte is contacted with at least one test site of a second sensor chip (1) in such a way that second ligands (7) present in the second analyte bind to the receptors (6a, 6b) located at the test site, wherein a value of the frequency of bindings of the second ligands to the receptors is measured and the concentration of the second ligands is determined on the basis of the measured value, and
- wherein the concentrations of the first and second ligands (7) differ from one another by at least two, in particular three and preferably 4 decades.

## Revendications

1. Puce de capteur (1) avec un support (2), sur la surface (3) duquel sont prévus des points d'essai (4a, 4b), sur lesquels sont immobilisés des récepteurs (6a, 6b) qui ont une capacité de liaison spécifique pour un ligand à détecter (7), dans laquelle les récepteurs (6a) sont liés sur la surface (3) sous la forme d'une monocouche et/ou de plusieurs couches moléculaires superposées l'une à l'autre, **caractérisée en ce qu'**il est prévu sur la surface (3) du support (2), en plus du premier point d'essai (4a) présentant les premiers récepteurs (6a) ou sur celuici, au moins un deuxième point d'essai (4b) auquel est fixé un réseau polymère (8), et **en ce que** sur et/ou dans le réseau polymère (8) sont immobilisés des deuxièmes récepteurs (6b) qui ont une capacité de liaison spécifique pour le ligand à détecter (7).

2. Puce de capteur (1) avec un support (2), sur la surface (3) duquel est prévu au moins un point d'essai (4b), auquel est fixé un réseau polymère (8) et/ou dans lequel sont immobilisés des récepteurs (6b) qui ont une capacité de liaison spécifique pour un ligand à détecter (7), **caractérisée en ce qu'**au moins un détecteur (5b) est intégré à la surface du support (2) pour détecter la liaison du ligand (7) sur les premiers récepteurs (6a) et/ou sur les deuxièmes récepteurs (6b) et **en ce que** le réseau polymère (8) est fixé directement ou indirectement sur le au moins un détecteur (5b) via au moins une couche des premiers récepteurs (6a).

3. Puce de capteur (1) selon la revendication 1 ou 2, **caractérisée en ce que** le réseau polymère (8) est un réseau polymère gonflant (8).

4. Puce de capteur (1) selon l'une quelconque des revendications 1 à 3, **caractérisée en ce que** le réseau polymère (8) présente des copolymères hydrophiles.

5. Puce de capteur selon l'une quelconque des revendications 1 à 4, **caractérisée en ce que** le réseau polymère (8) contient un polymère hydrosoluble préformé qui présente au moins un groupement photoréticulable, en particulier un groupement benzophénone.

6. Puce de capteur (1) selon l'une quelconque des revendications 1 à 5, **caractérisée en ce que** le réseau polymère (8) contient un polymère qui présente un groupement adhésif, en particulier de l'acide phosphonique.

7. Puce de capteur (1) selon l'une quelconque des revendications 1 à 6, **caractérisée en ce que** les premiers récepteurs (6a) et les deuxièmes récepteurs (6b) présentent les mêmes molécules.

8. Puce de capteur (1) selon l'une quelconque des revendications 1 à 7, **caractérisée en ce que** les premiers récepteurs (6a) et les deuxièmes récepteurs (6b) présentent différentes molécules.

9. Puce de capteur (1) selon l'une quelconque des revendications 1 à 8, **caractérisée en ce que** les premiers récepteurs (6a) sont immobilisés via un groupement silane sur la surface.

10. Puce de capteur (1) selon l'une quelconque des revendications 1 à 9, **caractérisée en ce que** la surface (3) du support (2) est recouverte en totalité, sur le premier point d'essai (4a) et le deuxième point d'essai (4b), par les premiers récepteurs (6a) agencés dans la monocouche et/ou dans les couches moléculaires superposées l'une à l'autre et **en ce que** le réseau polymère (8) est agencé sur les premiers récepteurs (6a) et est fixé indirectement par covalence via ceux-ci et/ou entre ceux-ci sur la surface (3) du support (2).

11. Puce de capteur (1) selon l'une quelconque des revendications 1 à 10, **caractérisée en ce que**, sur les points d'essai de la surface (3) du support (2), sont agencés des détecteurs (5a, 5b) pour détecter la liaison des ligands (7) avec les récepteurs (6a, 6b), qui forment une unité avec le support.

12. Puce de capteur (1) selon l'une quelconque des revendications 1 à 11, **caractérisée en ce que** le support (2) présente un matériau semi-conducteur et **en ce que** le au moins un détecteur (5b) est intégré au matériau semi-conducteur et **en ce que** le réseau polymère (8) contenant les récepteurs (6b) est agencé de préférence directement sur la surface du détecteur (5b).

13. Puce de capteur (1) selon l'une quelconque des revendications 1 à 12, **caractérisée en ce que** les détecteurs (5a, 5b) sont des détecteurs optiques (5a, 5b) pour enregistrer un rayonnement luminescent qui apparaît en fonction de la liaison des ligands (7) avec les récepteurs (6a, 6b).

14. Puce de capteur (1) selon l'une quelconque des revendications 1 à 13, **caractérisée en ce que** les détecteurs (5a, 5b) sont conformés pour mesurer un champ électrique qui apparaît en fonction de la liaison des ligands (7) avec les récepteurs (6a, 6b) et/ou une propriété électrique des ligands (7).

15. Puce de capteur (1) selon l'une quelconque des revendications 1 à 14, **caractérisée en ce qu'**il est prévu sur le support (2) plusieurs aménagements de capteurs, constitués respectivement des premiers récepteurs (6a) immobilisés sur le premier point d'essai (4a), du réseau polymère gonflant (8) agencé sur le deuxième point d'essai (4b) et des deuxièmes récepteurs (6b) qui y sont immobilisés, et **en ce que** les récepteurs (6a, 6b) d'au moins deux de ces aménagements ont une capacité de liaison spécifique pour différents ligands (7) et/ou présentent une densité d'occupation de surface différente.

16. Puce de capteur (1) selon l'une quelconque des revendications 1 à 15, **caractérisée en ce que** le support (2) est une bande souple, en particulier une feuille de polymère, et **en ce que** les aménagements de capteurs sont agencés dans le sens de l'extension de la bande de manière décalée l'un par rapport à l'autre.

17. Puce de capteur (1) selon l'une quelconque des revendications 1 à 16, **caractérisée en ce que** le support (2) est constitué d'un matériau synthétique, de préférence d'un matériau synthétique transparent.

18. Puce de capteur (1) selon l'une quelconque des revendications 1 à 17, **caractérisée en ce qu'**elle forme une paroi limitatrice d'une chambre de mesure et **en ce que** la chambre de mesure se présente de préférence sous la forme d'une chambre de mesure de débit (13) ayant au moins une ouverture d'entrée (15a, 15b) et au moins une ouverture de sortie (16a, 16b).

19. Puce de capteur (1) selon l'une quelconque des revendications 1 à 18, **caractérisée en ce que** la chambre de mesure présente au moins deux compartiments (14a, 14b) que jouxte respectivement au moins un aménagement de capteurs agencé sur la surface (3) du support (2).

20. Procédé de mesure d'une première concentration d'un premier ligand contenu dans un analyte à analyser et une deuxième concentration supérieure d'un deuxième ligand contenu dans l'analyte, **caractérisé en ce que** l'analyte est mis en contact avec les points d'essai d'une puce de capteur selon l'une quelconque des revendications 1 à 16 et **en ce que**, pour déterminer la première concentration, on mesure la fréquence des liaisons des premiers ligands avec les premiers récepteurs (6a) et pour déterminer la deuxième concentration, on mesure la fréquence des liaisons des deuxièmes ligands avec les deuxièmes récepteurs (6b).

21. Procédé selon la revendication 20, **caractérisé en ce que** la première concentration et la deuxième concentration diffèrent l'une de l'autre d'au moins une dizaine.

22. Procédé de mesure des concentrations de ligands contenus dans deux analytes à analyser, dans lequel au moins deux puces de capteur (1) de structure identique sont aménagées selon la revendication 2,
- dans lequel un premier analyte est mis en contact avec au moins un point d'essai d'une première puce de capteur (1) de manière que les premiers ligands (7) contenus dans le premier analyte se lient aux récepteurs (6a, 6b) se trouvant au point d'essai, une valeur de mesure pour la fréquence des liaisons des premiers ligands avec les récepteurs étant enregistrée et la concentration des premiers ligands étant déterminée à l'aide de la valeur de mesure,
- dans lequel un deuxième analyte est mis en contact avec au moins un point d'essai d'une deuxième puce de capteur (1) de manière que les deuxièmes ligands (7) contenus dans le deuxième analyte se lient aux récepteurs (6a, 6b) se trouvant au point d'essai, une valeur de mesure pour la fréquence des liaisons des deuxièmes ligands aux récepteurs étant enregistrée et la concentration des deuxièmes ligands étant déterminée à l'aide de la valeur de mesure, et
- dans lequel les concentrations des premiers et deuxièmes ligands (7) diffèrent d'au moins deux, en particulier de trois et de préférence de quatre dizaines l'une de l'autre.
